# EUROPEAN PATENT APPLICATION

(11) **EP 4 119 652 A1**
(43) Date of publication of application: **18.01.2023**
(21) Application number: 21185836.0
(22) Date of filing: 15.07.2021
(51) Int. Cl.: C12M 1/32, C12M 3/06, C12M 1/00

(54) **AN ASSAY PLATE ASSEMBLY TO ASSAY A CELL CULTURE MODEL**

(71) Applicant: Hooke Bio Ltd., V14XH92, Shannon, Co. Clare (IE)
(72) Inventor: Cliffe, Fionla, Co. Limerick, V94 DH6Y (IE); Lyons, Mark, Co. Clare, V94 F8N3 (IE); Madden, Conor, Limerick, Raheen (IE); Keady, Tara, Galway, H91 XP1H (IE); Devitt, Shane, Co. Clare, V95 HP7R (IE); Costello, Patrick, Co. Galway, Oranmore (IE); Ramesh, Sumir, Co. Clare, Shannon (IE)
(74) Representative: Purdylucey Intellectual Property

(57) **Abstract**

An assay plate assembly (1) to assay a cell culture model is described and comprises a first plate (2) having a plurality of well-receiving apertures (14) and a fluidic conduit (12, 13) configured to provide an assay fluid to the well receiving apertures, and a second plate (3) having a plurality of cell culture model assay wells (37) each comprising a lower well portion (41) with a well base (40) disposed in the second plate and an upper well portion (38) having an open top (39) defined by an annular sidewall that projects above a top surface of the second plate. The first and second plates are configured to nest together to form the assay plate assembly in which the upper well portion (38) of each cell culture model assay well (37) is disposed within a corresponding well-receiving aperture (14) of the first plate for receipt of fluid from the fluidic conduit (12, 13) through the open top of the cell culture model assay wells. The first plate is re-usable and the second plate is disposable.

## Description

### Field of the Invention

The present invention relates to an assay plate assembly to assay a 2D or 3-D cell culture model. Also contemplated is a method of assaying cell culture models that employ an assay plate assembly of the invention.

### Background to the Invention

During the process of drug discovery, test drugs need to be tested on cells to determine how they work and the effects of the drug on the cells. To date, the pharmaceutical industry has relied primarily on animal models and human cell line cultures that bear little resemblance to normal or disease human tissue, resulting in only one in every ten drugs making it through clinical testing. This high failure rate in clinical trials of drugs that make it through pre-clinical testing adds greatly to the cost of drug development.

Microtissues or organoids are tiny, self-organized three-dimensional tissue cultures that are derived from stem cells. Such cultures can be crafted to replicate much of the complexity of an organ, or to express selected aspects of it like producing only certain types of cells. Microtissues may be grown from a variety of precursor cell types including stem cells-cells that can divide indefinitely and produce different types of cells as part of their progeny. Scientists have learned how to create the right environment for the precursor cells so they can follow their own genetic instructions to self-organize, forming tiny structures that resemble miniature organs composed of many cell types. 3-D cell culture models such as organoids and microtissues can range in size from less than the width of a hair to 5mm.

Many scientists believe that using organoids/microtissues and other 3-D cell culture models have the capacity to be more accurate and physiologically relevant models than existing animal models and that using such 3-D cell culture models in pre-clinical testing of drugs will reduce the failure rate of drugs that enter clinical trials. These models will allow drug and vaccine manufacturers to bring products to market in a more timely and efficient manner. There is therefore a need for a technology to facilitate high throughput cell culture model screening that reproduces physiological conditions (e.g. temperature, CO2 and nutrients), facilitates fluid flow to the cell culture models, and allows cell culture models to be imaged.

It is an objective of the invention to overcome at least one of the above-referenced problems.

### Summary of the Invention

The objective is met by the provision of an assay plate assembly to assay 2-D or 3-D cell culture models such as organoids, microtissues and spheroids that comprises two plates that couple together to form the assay plate assembly. A first plate has apertures and an assay fluid supply conduit in fluidic communication with the apertures, and a second plate comprises assay wells that generally project proud of a surface of the second plate. The plates are configured to be nested together such that the assay wells of the second plate project into the corresponding apertures of the first plate whereby the wells receive assay fluid from the assay fluid supply conduit. The provision of the assay fluid supply system in a first plate which is separate from the plate with the assay wells allows the first plate to be re-used in subsequent assays, reducing the costs of the system as only the second plate needs to be replaced between assays.

In a first aspect, the invention provides an assay plate assembly to assay a cell culture model such as an organoid comprising;
a first plate having a plurality of well-receiving apertures and a fluidic conduit configured to provide an assay fluid to the well receiving apertures; and
a second plate having a plurality of cell culture model assay wells comprising an upper well portion that projects above an upper surface of the second plate,
wherein the first and second plates are configured to nest together to form the assay plate assembly in which the upper well portion of each assay well is disposed within a corresponding well-receiving aperture of the first plate for receipt of fluid from the fluidic conduit of the assay wells.

In another aspect, the invention provides an assay plate assembly to assay a cell culture model comprising
a first plate having an upper part, a lower part, and a plurality of well-receiving apertures formed in the lower part, and a fluidic conduit configured to provide an assay fluid to the well receiving apertures; and
a second plate having a lower part, an upper part, and a plurality of assay wells each comprising a lower well portion with a well base disposed in the upper part of the second plate and an upper well portion having an open top that projects above an upper surface of the second plate,
wherein the first and second plates are configured to detachably attach together to form the assay plate assembly in which the upper well portion of each assay well nests within a corresponding well-receiving aperture of the first plate for receipt of fluid from the fluidic conduit through the open top of the assay wells.

In any embodiment, the first plate is re-usable and the second plate is disposable.

In any embodiment, the first plate comprises at least one, and typically a plurality, of fluid control valve(s) configured to control the flow of fluid to the assay wells through the fluidic conduit. In any embodiment, the fluid control valve is selected from an electrically actuated fluid control valve (e.g. a Burkert valve) and a mechanically actuated fluid control valve.

In any embodiment, the lower part of the second plate comprises a light transparent material configured to allow imaging of the assay wells by an imaging device from underneath the wells.

In any embodiment, the base of each assay well is formed a light transparent material.

In any embodiment, the light transparent material is gas permeable.

In any embodiment, the sidewalls of each assay well is formed from a material that provides structural rigidity to the plate, for example an acrylic material.

In any embodiment, the lower part of the second plate comprises a plurality of well imaging sections comprising light transparent material each having an upper section that forms the base of the well and a lower section that tapers outwardly towards a base of the second plate.

In any embodiment, the plurality of well imaging sections comprising light transparent material are mounted on a planar base typically formed of light transparent material.

In any embodiment, the light transparent material comprises PDMS.

In any embodiment, the second plate comprises a section with a shaped recess dimensioned to receive the well imaging sections.

In any embodiment, the assay plate assembly comprising a base plate (optionally detachably) attached to the lower part of the second plate having a plurality of through-apertures positioned to allow imaging of the base of the assay wells by an imaging device through the well imaging sections.

In any embodiment, the base plate is re-usable.

In any embodiment, at least one and typically all of the assay wells comprise a sidewall that tapers inwardly from the open top towards the well base.

In any embodiment, the well base of one or more assay wells has a diameter D1 and the open top of the assay wells has a diameter D2, wherein a ratio of diameter D1 to D2 is 3:6 to 5:6.

In any embodiment, one or more of the assay wells has a concave base.

In any embodiment, the or each assay well has a depth of 1 to 7 mm.

In any embodiment, an open top of the or each assay well has diameter of 1 to 3.5 mm.

In any embodiment, the base of the or each assay well has diameter of 1 to 3.5 mm.

In any embodiment, the or each assay well has volume of 5-100 µL.

In any embodiment, a lower part of the first plate comprises a recess dimensioned to receive the second plate, or an assembly of the second plate and base plate, in a nested configuration.

In any embodiment, the base plate comprises an annular flange comprising a plurality of through apertures configured to receive fixing screws to fix the base plate to the first plate.

In any embodiment, the first plate comprises a recess formed in an upper surface thereof and a top layer of resiliently deformable material dimensioned to nest in the recess.

In any embodiment, the resiliently deformable material is a silicone material.

In any embodiment, the recess comprises an elongated groove and the fluidic conduit of the first plate is defined along at least a part of its length between the elongated groove and the top layer of resiliently deformable material.

In any embodiment, the elongated groove comprises a localised depression. The purpose of the depression is to allow a fluid control valve in the form of a pin close the fluidic conduit. The pin is generally disposed above the first plate and is actuatable to push against the resiliently deformable material into the depression to close the fluidic conduit.

In any embodiment, the first plate comprises a plurality of fluidic conduits.

In any embodiment, the elongated groove of the or each fluidic conduit comprises a plurality of spaced-apart pin-receiving depressions.

In any embodiment, the plurality of assay wells are arranged in a N x M grid assembly (N rows and M columns) in which N and M are each, independently. a whole number greater than or equal to 2, 3, 4, 5, 6, 7, 8, 9, or 10.

In any embodiment, the first plate comprises a first fluid supply conduit configured to supply assay fluid to at least one column of assay wells in series (e.g. in the case of 3x3 grid of wells, the first fluid supply conduit fluidically connects an assay fluid reservoir with a first well in the column, fluidically connects a second well in the column with the first well, and fluidically connects a third well in the column with the second well).

In any embodiment, the first fluid supply conduit is configured to supply assay fluid to a plurality or all of the columns of assay wells in series.

In any embodiment, the first plate comprises a second fluid supply conduit configured to supply assay fluid to at least one row of assay wells in series (e.g. in the case of 3x3 grid of wells, the first fluid supply conduit fluidically connects an assay fluid reservoir with a first well in the row, fluidically connects a second well in the row with the first well, and fluidically connects a third well in the row with the second well).

In any embodiment, the second fluid supply conduit is configured to supply assay fluid to a plurality or all of the columns of assay wells in series.

In any embodiment, the first and/or second fluidic conduits comprise a single upstream conduit that branches into a plurality of conduit branches each supplying a column or row of wells.

In any embodiment, each conduit branch comprises a fluid control valve disposed upstream of the wells.

In any embodiment, the first plate comprises a plurality of fluid control valves configured to limit fluid flow across the wells in an X direction or in a Y-direction.

In any embodiment, the first plate comprises a series of valves that when actuated allow flow of assay fluid along the column of wells (X-direction) and prevent fluid flow along the rows of wells (Y-direction). The valves in the series are typically disposed between adjacent wells in a row of wells.

In any embodiment, the first plate comprises a series of valves that when actuated allow flow of assay fluid along the rows of wells (Y-direction) and prevent fluid flow along the columns of wells (X-direction). The valves in the series are typically disposed between adjacent wells in a column of wells.

In another aspect, the invention provides an assay plate (e.g. forming part of an assay plate assembly of the invention) and comprising a plate body having a lower part, an upper part, and a plurality of assay wells each comprising a lower well portion with a well base disposed in the upper part of the second plate and an upper well portion with an open top defined by an annular sidewall that projects above an upper surface of the second plate.

In any embodiment, the lower part of the second plate comprises a light transparent material configured to allow imaging of the assay wells by an imaging device from underneath the wells.

In any embodiment, the base of each assay well is formed a light transparent material.

In any embodiment, the light transparent material is gas permeable.

In any embodiment, the sidewalls of each assay well is formed from an acrylic material.

In any embodiment, the lower part of the second plate comprises a plurality of well imaging sections comprising light transparent material each having an upper section that forms the base of the well and a lower section that tapers outwardly towards a base of the second plate.

In any embodiment, the plurality of well imaging sections comprising light transparent material are mounted on a planar base typically formed of light transparent material.

In any embodiment, the light transparent material is gas permeable. In any embodiment, the light transparent material comprises or consists of PDMS. Other light transparent materials include cyclicolefin copolymer (COC), perfluoropolyethers (PFPEs), polyurethane, Flexdym, and polylactic acid (PLA)

In any embodiment, the second plate comprises a section comprising a shaped recess dimensioned to receive the well imaging sections.

In any embodiment, the assay plate comprising a base plate (optionally detachably) attached to the lower part of the plate body having a plurality of through-apertures positioned to allow imaging of the base of the assay wells by an imaging device through through-apertures of the base plate and the transparent well imaging sections of the plate body. In any embodiment, the base plate is re-usable.

In any embodiment, at least one and typically all of the assay wells comprise a sidewall that tapers inwardly from the open top towards the well base.

In any embodiment, the well base of one or more assay wells has a diameter D1 and the open top of the cell culture model assay wells has a diameter D2, wherein a ratio of diameter D1 to D2 is 3:6 to 5:6.

In any embodiment, one or more of the assay wells has a concave base.

In any embodiment, the or each assay well has a depth of 1 to 7 mm.

In any embodiment, an open top of the or each assay well has diameter of 1 to 3.5 mm.

In any embodiment, the base of the or each assay well has diameter of 1 to 3.5 mm.

In any embodiment, the or each assay well has volume of 5-100 µL.

In any embodiment, the base plate comprises an annular flange comprising a plurality of through apertures configured to receive fixing screws to fix the base plate to the first plate.

In any embodiment, the plurality of assay wells are arranged in a N x M grid assembly (N rows and M columns) in which N and M are each, independently. a whole number greater than or equal to 2, 3, 4, 5, 6, 7, 8, 9, or 10.

In another aspect, the invention provides a system comprising:
an assay plate assembly according to the invention; and
a well imaging system configured to image the wells of the assay plate assembly from underneath the assay plate assembly.

In any embodiment, the system comprises a fluid control module comprising at least one pin and an actuation system coupled to the at least one pin configured to move the pin between a first configuration in which the pin is spaced apart from the first plate and a second configuration in which the pin is advanced into the fluidic conduit of the first plate to close the fluidic conduit.

In any embodiment, the fluid control module comprises at least two pins and the actuation system is configured to independently actuate each pin.

In any embodiment, a first pin is disposed on a first pin plate and a second pin is mounted on a second pin plate, in which the first pin plate comprises an aperture configured to receive the second pin when the second pin is moved by the actuation system from the first configuration to the second configuration.

In any embodiment, the first pin plate comprises a plurality of first pins and the second pin plate comprises a plurality of second pins, in which the first pin plate comprises a plurality of apertures aperture configured to receive the corresponding second pins.

In any embodiment, the first plate comprises a recess formed in an upper surface thereof and a top layer of resiliently deformable material dimensioned to nest in the recess, in which the recess comprises an elongated groove and the fluidic conduit of the first plate is defined along at least a part of its length between the elongated groove and the top layer of resiliently deformable material, wherein the pin of the fluid control module is configured to press against the resiliently deformable material to close the fluidic conduit.

In any embodiment, the elongated groove of the fluidic conduit comprises a depression, wherein the pin is configured to press the resiliently deformable material into the depression to close the fluidic conduit.

In any embodiment, the assay plate assembly comprises a grid on N x M assay wells, wherein the first plate comprises:
In another aspect, the invention provides a method of assaying a cell culture model that employs an assay plate assembly according to invention or a system according to the invention, the method comprising the steps of:
   adding a cell culture model or a precursor cell into an assay well of the second plate;
   coupling the second plate with the first plate such that the upper well portions of the assay wells of the second plate are nested and fluidically sealed within corresponding well-receiving apertures of the first plate;
   providing assay fluid to the assay well containing the cell culture model precursor cell through the fluidic conduit of the first plate; and
   assaying the response of the cell culture model to the assay fluid.
In any embodiment, the method comprises adding a cell culture model or precursor cell into a plurality of assay wells of the second plate, providing assay fluid to the assay wells containing the cell culture model or precursor cell through the fluidic conduit of the first plate, and assaying the response of the cell culture model to the assay fluid.
In any embodiment, the method comprises a step of growing at least one cell culture model *in-situ* within an assay well after the assay plate assembly has been assembled, in which the assay fluid typically comprises an culture medium.
In any embodiment, the method comprises a step of assaying the response of the cell culture model to a test compound, in which the assay fluid comprises the test compound.
In any embodiment, the response of the cell culture model that is assayed is cell viability.
In any embodiment, the assay fluid comprises a reagent configured to allow cell viability of the cell culture model be determined by imaging the well.
In any embodiment, the response of the cell culture model that is assayed is protein expression. This may be determined by sampling the fluid in the well and assaying for protein expression by the cell culture model.
In any embodiment, a first cell culture model or precursor cell is added into a first assay well and a second cell culture model or precursor cell is added into a second assay well.
In any embodiment, the assay plate assembly comprises a column of wells fluidically connected in series in which at least one of the wells of the column contains a cell culture model having a first tissue type (for example, the first well may contain a liver organoid) and at least one other of the wells of the column contains a cell culture model having a second tissue type (for example, the second well may contain a liver organoid).
In any embodiment, the method comprises providing an assay fluid containing a test compound to a first well containing a cell culture model having a first tissue type, and then providing fluid from the first well containing the test compound to a second well containing a cell culture model having a second tissue type.
In any embodiment, the assay plate assembly comprises at least nine assay wells arranged in a grid comprising N columns and M rows in which N and M are each independently 3 or greater.
In any embodiment, the assay plate assembly comprises a first fluidic conduit configured to supply assay fluid to one or more of the N columns of wells in which the method comprises supplying assay fluid to the wells of one or more of the columns of wells in series.
In any embodiment, the assay plate assembly comprises a second fluidic conduit configured to supply assay fluid to one or more of the M rows of wells in which the method comprises supplying assay fluid to the wells of one or more of the rows of wells in series.
In any embodiment, the method comprises additional steps of:
   detaching the second plate from the first plate after the first assay;
   adding a cell culture model or precursor cell into an assay well of a replacement second plate;
   coupling the replacement second plate with the first plate such that the upper well portions of the assay wells of the replacement second plate are nested and fluidically sealed within corresponding well-receiving apertures of the first plate;
   providing assay fluid to the assay well containing the cell culture model or precursor cell through the fluidic conduit of the first plate; and
   assaying the response of the cell culture model or to the assay fluid.
In any embodiment, the cell culture model is selected from an organoid, and/or a microtissue and/or a spheroid.

In another aspect, the invention provides a method of fabricating a second plate of the assay plate assembly of the invention, the method comprising the steps of:
providing a plate have an upper surface, a lower surface, and a plurality of through apertures each comprising an upper aperture section defined an annular sidewall projecting proud of the upper surface of the second plate and a lower aperture section with an open base disposed in the plate;
placing a mould having a plurality of well-shaped projections on top of the plate such that the projections nest within corresponding through apertures of the plate, wherein the projections are dimensioned to extend into and nest snugly in the upper aperture section and a distal tip of each projection extends into the lower aperture section;
casting a light transparent material in the lower aperture sections of the plate with the mould *in-situ;* and
removing the mould to provide a plate comprising a plurality of wells each having a base formed of a light transparent material and a well imaging sections comprising light transparent material disposed under each well.

In any embodiment, the lower surface of the plate comprises a recess in which the open base of each through aperture is disposed within the recess, in which the casting step comprises casting the light transmissible material in the recess and lower aperture sections of the plate with the mould in-situ.

In any embodiment, the lower aperture section is frustoconical.

In any embodiment, the distal tip of each projection is convex.

In any embodiment, the method comprises a step of attaching a base plate to the lower surface of the plate, in which the base plate comprises a plurality of through-apertures configured to allow imaging of the base of the wells by an imaging device through the through-apertures and imaging sections of the plate.

Other aspects and preferred embodiments of the invention are defined and described in the other claims set out below.

### Brief Description of the Figures

**FIG. 1** is a top plan view of an assay plate of the invention.
**FIG.2** is an end elevational view of the assay plate of Figure 1.
**FIG. 3** is a detailed view of an assay well and two fluidic conduits of the assay plate of Figure 1.
**FIG.4** is a detailed view of a depression forming part of the second recess of the assay plate of Figure 1.
**FIG. 5** is a sectional view of part of the assay plate of Figure 1 showing adjacent assay wells and fluidic conduit with a depression fluidically connecting the top of thr wells.
**FIG. 6** is a sectional view taken along the lines A-A of Figure 1.
**FIG.7A** is a perspective view from above of a second plate part forming part of one embodiment of an assay plate of the invention.
**FIG. 7B** is a perspective view from below of the second plate part of Figure 7A.
**FIG.7C** is a side elevational view of the second plate part of Figure 7A.
**FIG.8A** is a perspective view from above of a base plate part forming part of one embodiment of the assay plate of the invention.
**FIG. 8B** is a perspective view from below of the base plate part of Figure 8A.
**FIG.8C** is a side elevational view of the base plate part of Figure 8A.
**FIG. 9** is a sectional view of the assay plate of the invention showing the assay wells and first section of a fluidic conduit disposed in the first recess, second recess with second section of the fluidic conduit, third section of the fluidic conduit and fluid recirculation conduit.
**FIG. 10** is a perspective view showing the assay plate system of the invention including plate body with first and second recesses and a first fluid control module positioned over the first recess and second fluid control module positioned over the second recess with pins engaged into the depressions of the second recess.
**FIG. 11A** is a side elevational view of an assay plate system of Figure 10 showing the actuation system for the first fluid control module positioned and second fluid control module.
**FIG. 11B** is a sectional view taken along the line E-E of Figure 11A.
**FIG. 11C** is a sectional view taken along the line F-F of Figure 11A.
**FIG. 12A** is an end elevational view of an assay plate system of Figure 10 showing the actuation system for the first fluid control module positioned and second fluid control module.
**FIG. 12B** is a sectional view taken along the line E-E of Figure 12A.
**FIG. 12C** is a sectional view taken along the line F-F of Figure 12A.
**FIG. 13** is a sectional elevational view of the assay plate assembly of the invention showing an assay fluid supply and recirculation system.
**FIG. 14** is an illustration of the second plate and base coupled together during formation of the PDMS well imaging section of the second plate, with the casting mould comprising well-shaped projections inserted into the open top of the wells.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

The term "cell culture model" refers to a 3-D cell culture model or a 2-D cell culture model. As used herein, the term "3-D cell culture model" refers to a miniaturized and simplified version of an organ produced in vitro in three dimensions that shows realistic micro-anatomy. Examples include organoids, microtissues and spheroids. Organoid and microtissues are derived from one or a few cells from a tissue, embryonic stem cells or induced pluripotent stem cells, which can self-organize in three-dimensional culture owing to their self-renewal and differentiation capacities. Such 3-D cell culture models typically have a maximum dimension of 50-1500 µm.

Examples of 3-D cell culture models include liver, hepatic, pancreatic, epithelial, kidney, cardiac, retinal, blastoid, glioblastoma, thyroid and testicular organoids.

As used herein, the term "cell culture model precursor" or "precursor" refers to a cell or composition of cells capable of being cultured to form a 3-D or 2-D cell culture model. The precursor may be a cell from a specific tissue, a cell line, a patient cell or tissue sample, an embryonic stem cell, or an induced pluripotent stem cell. The assay plate system of the invention may be used to assay cell culture models such as organoids and to grow cell culture model from precursor cells. Growth generally comprises placing a suitable precursor cell into a well, adding assay fluid (which may be cell culture fluid containing agents to promote the growth of the desired cell culture model), and recirculation of the assay fluid to the well or wells.

As used herein, the term "assay plate assembly" refers to an assay plate formed by attaching together a first and second plate, and optionally a base plate, and comprising one or more cell culture model receiving wells and one or more fluidic conduits configured to supply assay fluid (that generally includes a test compound) to the wells. The plate assembly is generally planar. The wells of the second plate have an upper well part that projects proud of a surface of the second plate. The first plate comprises apertures for receiving the wells of the second plate in a fluidically tight manner. A sealing o-ring may be provided to seal with a well in a well receiving aperture. Generally, a base of the first plate comprises a recess to receive the second plate in a nested configuration. The first plate may be formed from a metal or alloy or polymer, for example an acrylic, and the second plate is generally formed a material such as acrylic that confers structural rigidity to the plate and a light transparent and gas permeable material (e.g. PDMS).

As used herein, the term "disposable" as applied to the second plate means that the second plate is not re-usable and is detached from the first plate and disposed of after use, allowing the first plate to be re-used with a replacement second plate.

As used herein, the term "light transparent material" refers to a material that is transparent. Sections of the lower part of the second plate, including the base of the wells, is generally formed from a light transparent material allow the cell culture model in the wells to be imaged with an imaging device from below the second plate. The light transparent material is generally a polymeric material suitable for melting and casting. The light transparent material may be PDMS, cyclicolefin copolymer (COC), perfluoropolyethers (PFPEs), polyurethane, Flexdym, polylactic acid (PLA). In any embodiment, the light transparent material is gas permeable.

As used herein, the term "N x M grid assembly" as applies to the wells of the second plate refers to a plurality of wells arranged in a grid format comprising M columns and N rows. Generally M and N are each, independently, a whole number greater than 2, for example 3, 4, 5, 6, 7, 8, 9, 10, 11 or 12.

As used herein, the term "well imaging system" refers to an imaging device configured to image the wells of the plate assembly, generally from a position beneath the plate assembly. Examples of imaging devices include microscopes that can undertake white light and fluorescent imaging.

As used herein, the term "fluid control module" refers to a part of the system of the invention that is actuatable to open and close a fluidic conduit of the first plate. The module comprises at least one pin and an actuation system for the pin to move the pin from a first position spaced apart from the plate assembly to a second position in which the pin causes the fluidic conduit to close. The fluid control module generally has a plurality of pins, for example at least 8, 18, 32, or 50. Generally the pins comprise a first set of pins that actuate together, and a second set of pins that actuate together, in which the actuation system is configured to actuate the first set of pins independently of the second set of pins. In one embodiment, the first set of pins are mounted to a first plate, the seconds set of pins are mounted to a second plate disposed above the first plate, and the first plate comprises a plurality of apertures corresponding to the second set of pins and configured to allow the second set of pins project through the first plate when the second plate is moved towards the first plate. The actuation system is operatively coupled to the first and second plates and configured to move each plate independently towards the top surface of the first plate.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

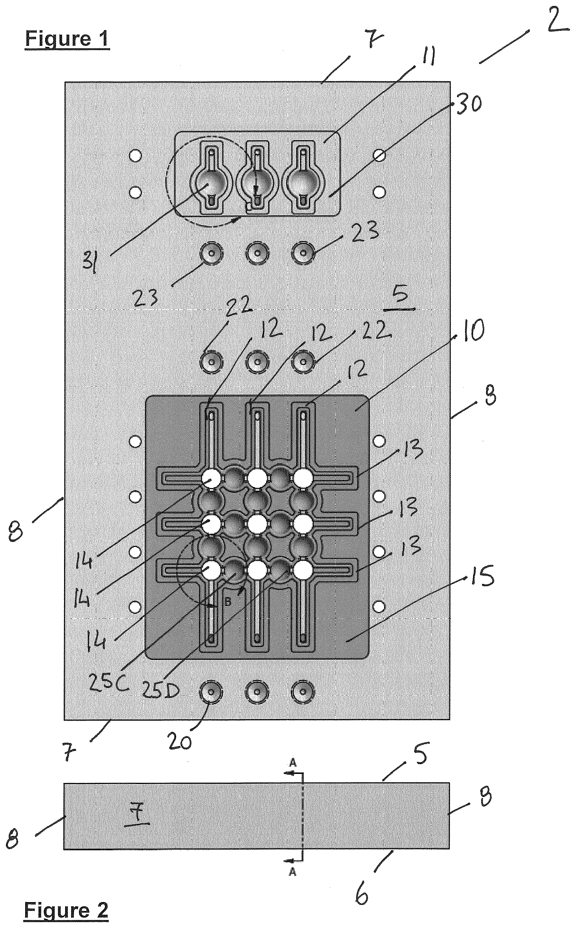

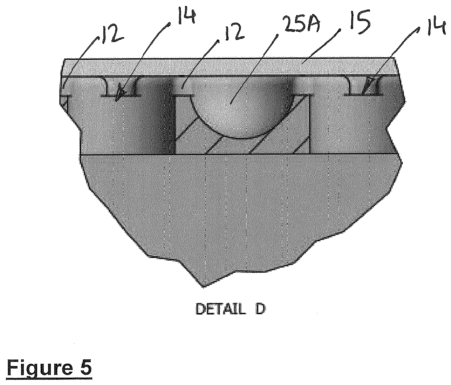

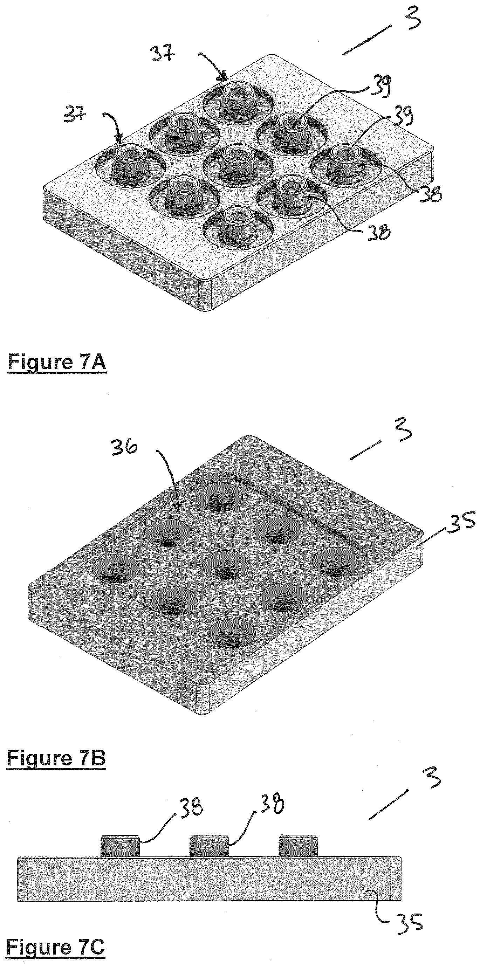

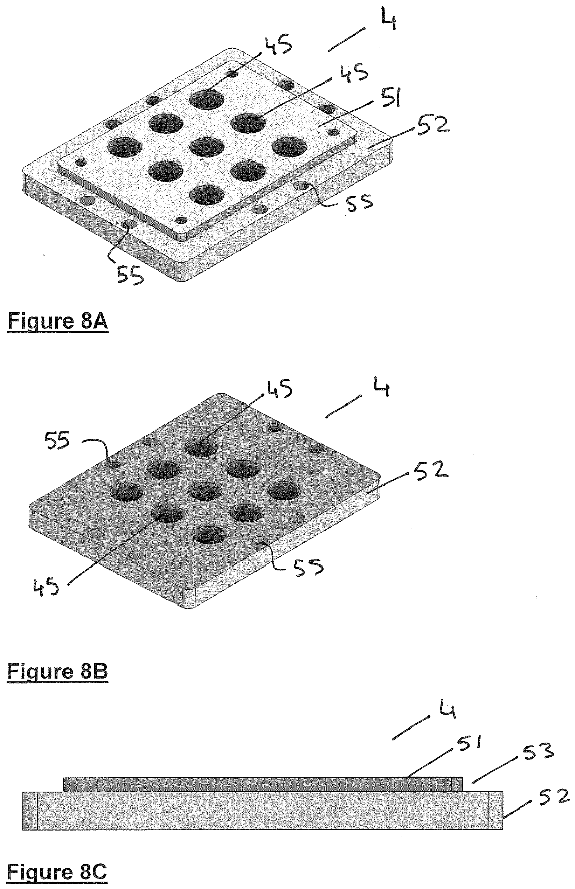

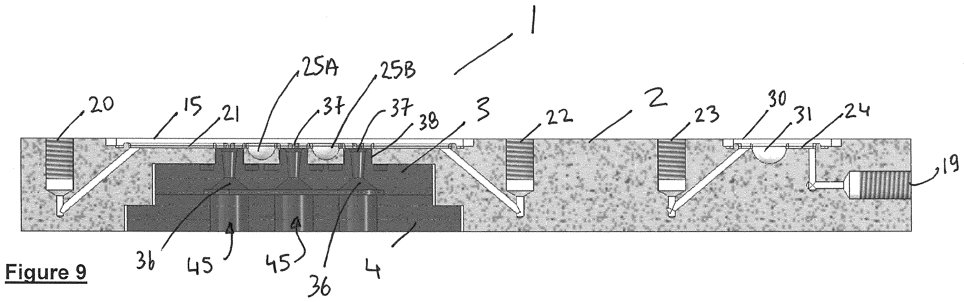

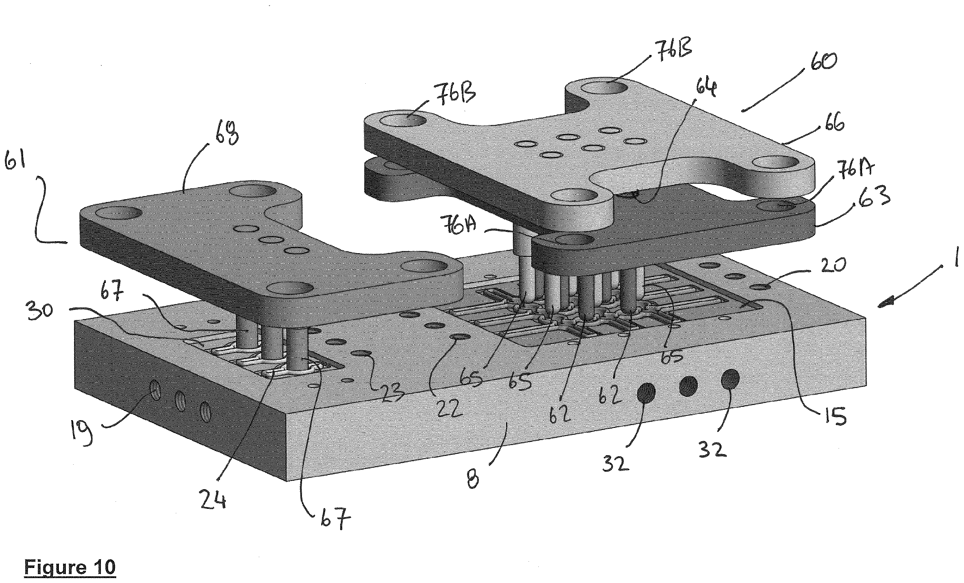

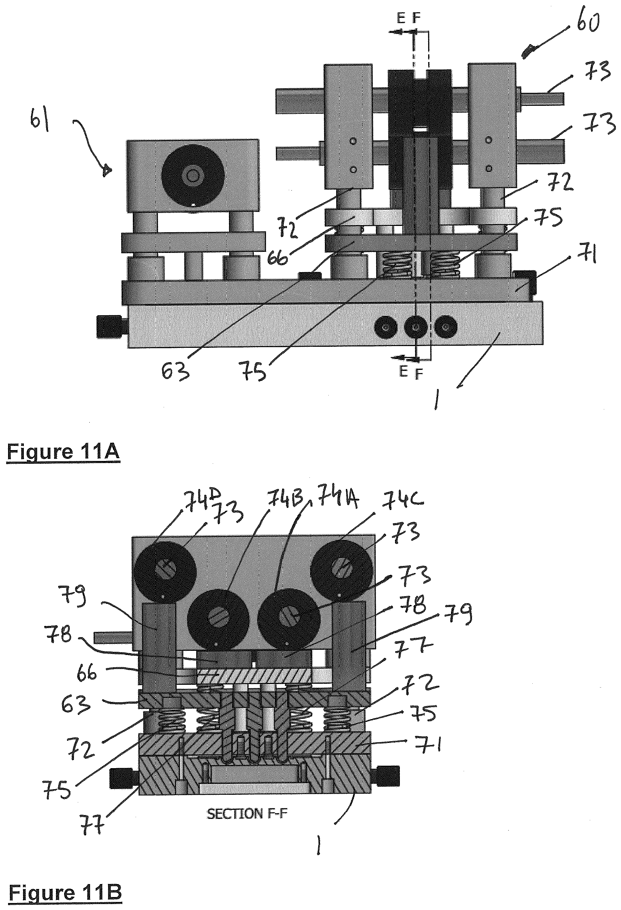

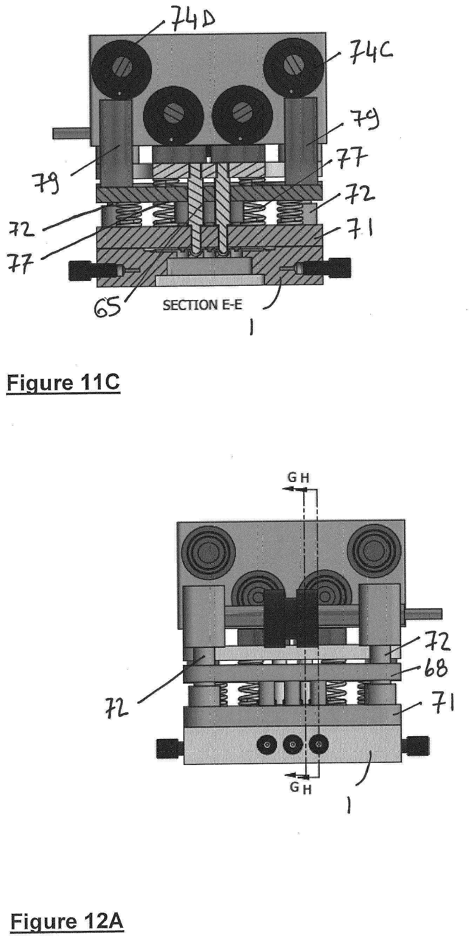

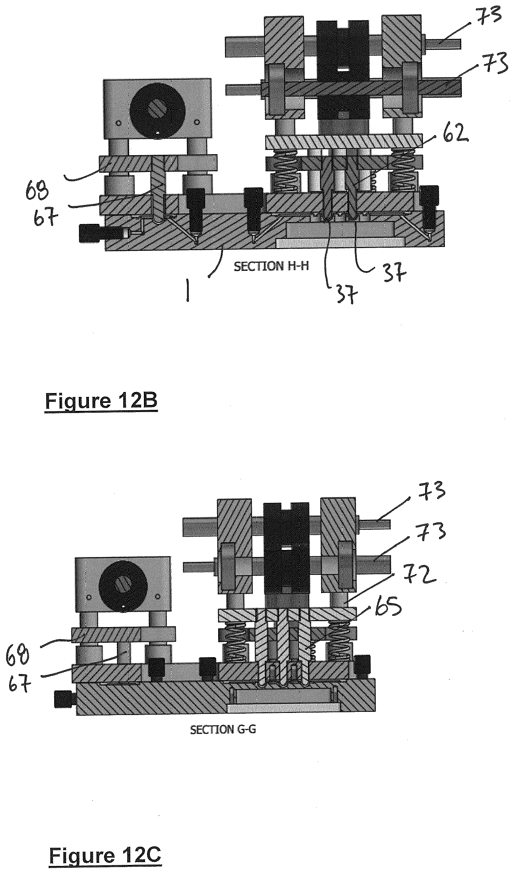

Referring to the drawings, an assay plate system of the invention is described in detail. In the embodiment describes, the system comprises an assay plate 1 formed from three parts that nest together to form the plate body, namely a first (upper) plate part 2 illustrated in Figures 1 to 6, a second plate part 3 illustrated in Figures 7A to 7C, and a base plate part 4 illustrated in Figures 8A to 8C. The provision of wells in the second plate part allows the second plate part to be disposable, allowing re-use of the first plate part which does not come into direct contact with the cell culture model that is being assayed. Figure 9 illustrated the assembled plate body. Figure 10 illustrates the first fluid control module disposed over the assay wells in the plate, and Figure 11 illustrates an assay fluid recirculation system comprising the first fluid control module disposed over the assay wells disposed in the first recess and a second fluid control module disposed over fluid pumping wells disposed in a second recess in the plate. Figure 12 illustrates one full fluidic circuit comprising a fluidic conduit and fluid recirculation conduit, and a fluid inlet and fluid outlet. It will be appreciated that the plate body does not need to be formed in separate parts but may be formed from a single plate body.

Referring to Figures 1 to 6, the first plate part 2 is described and comprises a planar plate having a top surface 5, lower surface 6, end walls 7 and sidewalls 8. The top surface 5 has a first recessed section 10 and a second recessed section 11. The first recessed section 10 comprises three first fluidic conduits 12 (Y-conduits) and three second fluidic conduits 13 (X-conduits) that intersect at through apertures 14. The apertures receive wells provided on a second plate part as described below during assembly of the plate such that the top of the wells is provided on the same plane as the fluidic conduits. When assembled the first recessed section has nine wells arranged in a 3 x 3 grid array. The fluidic conduits are defined along part of their length by elongated grooves formed in the top surface of the first recessed section 10 and a silicone layer 15 (not visible) that covers the top of the recess, fluidically sealing the recess and forming an upper surface of the fluidic conduits. In this manner, the fluidic conduits can be deformed and pinched closed using pins of a fluid control module as described in more detail below.

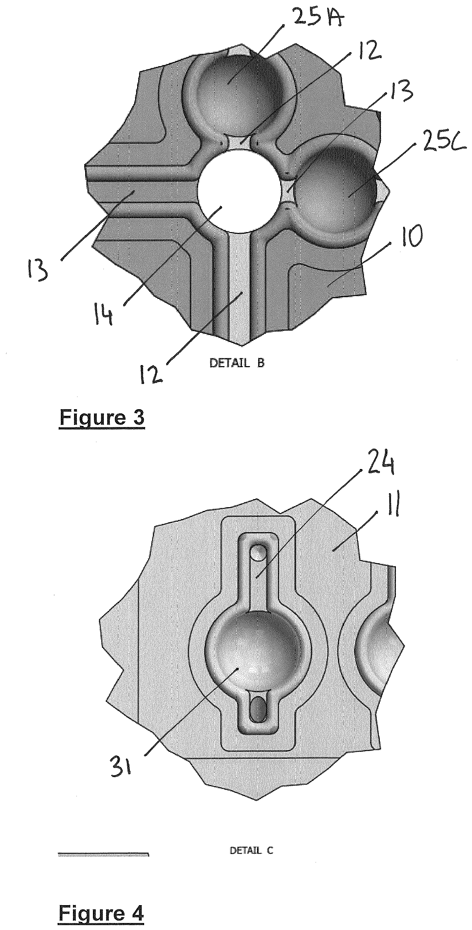

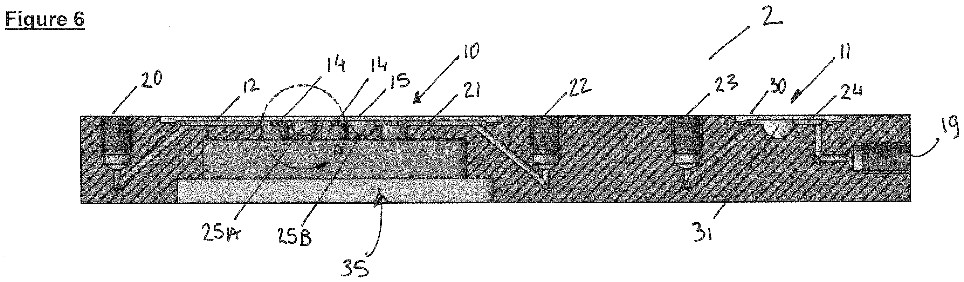

Each of the first fluidic conduits 12 (Y-conduits) has a fluid inlet 20 on the top of the plate, a first section 21 disposed in the first recessed section 10, an outlet 22 on the top of the plate, an inlet 23 on the top of the plate, a second section 24 disposed in the second recessed section 11, and a fluid outlet 19 disposed in the end wall 7 (See Figure 6). The first 21 section of the fluidic conduit provides fluidic communication to a column of three wells (disposed in the apertures 14 when the plate is assembled, as illustrated in Figure 9) in series and includes a first depression 25A disposed between the first and second apertures and a second depression 25B disposed between second and third apertures. Referring specifically to Figure 3, a section of the first recessed section 10 of the plate is illustrated showing a first fluidic conduit 12 intersecting with a second fluidic conduit 13 at a well-receiving aperture 14 and a depression 25A disposed between the first and second wells in the column of wells. The depression 25A has a width and depth greater than the width and depth of the fluidic conduits allowing a pin forming part of a fluid control module to press the overlying silicone layer into the depression to close the first fluidic conduit (as described in more detail below).

The second section 24 of the first fluidic conduits 12 is provided in the second recessed section 11 and defined by an elongated groove and a second silicone layer 30 that covers the top of the recess 11, fluidically sealing the recess and forming an upper surface of the second section of the fluidic conduits. The second section 24 also includes a fluid pumping well 31 that in use receives a pin forming part of a second fluid control module to press the overlying silicone layer into the well to pump fluid in a circuit around the plate.

Each of the three second fluidic conduits 13 (X-conduits) comprises a fluid inlet 32 disposed in a sidewall 8 of the plate, a fluid outlet (not shown) disposed in an opposite sidewall of the plate, and a central section disposed in the recessed section of the plate 10 (defined as described for the first fluidic conduit by an elongated groove and cover of silicone) that provides fluidic communication to a column of three wells (disposed in the apertures 14 when the plate is assembled, as illustrated in Figure 9) in series. Each of the second fluidic conduits 13 includes a first depression 25C disposed between the first and second apertures in the row and a second depression 25D disposed between second and third apertures in the row.

Referring to Figure 6, the lower surface of the plate comprises a stepped recess 35 dimensioned to receive the second plate part 3 and base plate part 4. The assembled plate with the second plate part 3 and base plate part 4 nested in the recess 35 is illustrated in Figure 9.

Referring to Figures 7A to 7C, the second plate part 3 has a rectangular planar plate body comprising a first plate section 35 formed of acrylic and a lower well imaging section 36 formed of light transparent and gas diffusing PDMS. Nine wells 37 are provided each having an upper well section 38 projecting proud of the top surface of the plate and a lower well section 41 formed in the plate. Each well has a depth of about 6.75 mm with an open top 39 having a diameter of about 2.89 mm and a concave base 40 having a diameter across the top of the concave part of about 2.04 mm. The sidewalls of each well 37 are defined by the first plate section 35. The PDMS well imaging section 36 has a planar base and a plurality of frustoconical sections 43 that extend upwardly with inwardly tapering sidewalls from the planar base to form the concave base 40 of the wells. The base plate has apertures 45 in the base that are aligned with the frustoconical PDMS sections 36 to allow the wells to be viewed with an imaging device (not shown) disposed underneath the plate assembly 1. The PDMS also allows for gas diffusion into the wells.

Referring to Figures 8A to 8C, the base plate 4 is illustrated and comprises a planar rectangular plate having a top part 51, bottom part 52, stepped sidewall 53, and nine apertures 45 extending through the plate from the top surface to the bottom surface. The top part 51 is dimensioned to align with the base of the second plate part 3 when the second plate part and base plate part are coupled together. Referring to Figure 3, the bottom part comprises holes 55 to receive fixing screws for fixing the base plate part 4 in the recess 35 of the first plate part 2.

Referring to Figure 10, an assay plate system according to one embodiment of the invention is illustrated in which parts described with reference to the previous embodiments are assigned the same reference numerals. The system comprises an assay plate 1 as described with reference to Figures 1 to 9, a first fluid control module 60 positioned above the 3 x 3 grid array of wells 37 disposed in the first recessed section 10 of the plate, and a second fluid control module 61 positioned above the second recessed section 11 of the plate. The actuation system for the fluid control modules is removed for clarity. The first fluid control module 60 comprises a first set of pins 62 attached to a first plate 63 having a plurality of apertures 64, and a second set of pins 65 that are longer than the first set of pins attached to a second plate 66 disposed above the first plate 63, with the second set of pins projecting through the apertures of the first plate 63. The second fluid control module 61 comprises three pins 67 attached to a third plate 68.

The first set pins 62 are positioned over the depressions 25A, 25B in the first fluidic conduits 12 and the second set of pins 65 are positioned over the depressions 25C, 25D in the second fluidic conduits 13. A plate actuation means (illustrated in Figures 11 and 12) is operatively coupled to each plate and configured to move each plate independently towards the top of the assay plate 1. When the second plate 66 is moved towards the assay plate 1 and the first plate 63 is not moved, the second set of pins 65 are depressed pushing the silicone cover into the depression 25C, 25D in the second fluidic conduits 13 closing those conduits and preventing assay fluid moving across the rows of wells of the plate in a X-direction, with the result that flow of assay fluid is channelled across the columns of wells via the first fluidic conduits 12 in a Y-direction. Similarly, when the first plate 63 is moved towards the assay plate 1 and the second plate 66 is not moved, the first set of pins 62 are depressed pushing the silicone cover into the depressions 25A, 25B of closing the first fluidic conduits 12 and preventing assay fluid moving across the wells of the plate in a Y-direction, with the result that flow of assay fluid is channelled across the column of wells via the X-direction conduits.

Figures 11A to 11C and 12A to 12C illustrate the actuation system for the first fluid control module 60 and second fluid control module 61 in more detail. The actuation system for the first fluid control module 60 comprises a frame 70 having a base plate 71 with apertures, upright supporting arms 72 and four axles 73 mounted between the arms. Each axle supports an eccentrically mounted camwheel 74. The first plate 63 is mounted to the arms 72 which are received by apertures 76A to allow vertical sliding movement on the plate of the arms and is spaced apart from the base plate 72 by first springs 75. The second plate 66 is similarly mounted to the arms received in apertures 76B for vertical sliding movement and spaced apart from the base plate 72 by second springs 77. The springs bias the plates away from the assay plate 1. The camwheels 74A and 74B are operably connected to the second plate 66 by cam followers 78 so that when the cam wheels rotate the second plate is urged downwardly pushing the second set of pins 65 into engagement with the silicone cover and fluidic conduits as illustrated in Figure 11C and 12C. The camwheels 74C and 74D are operably connected to the first plate 63 by cam followers 79 so that when the cam wheels rotate the first plate is urged downwardly pushing the first set of pins 62 into engagement with the silicone cover and fluidic conduits as illustrated in Figure 11B and 12B. Although the springs and cam followers are not illustrated second fluid control module 61 is of broadly the same construction as the first fluid control module.

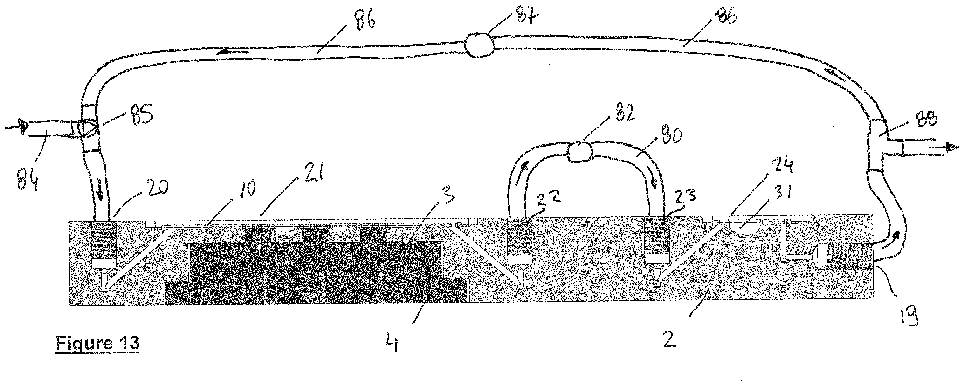

Referring to Figure 13, the fluid recirculation system of the assay plate 1 is described in more detail. One first fluidic conduit is illustrated and comprises a fluid inlet 20 on the top of the plate, a first section 21 disposed in the first recessed section 10, an outlet 22 on the top of the plate, an inlet 23 on the top of the plate, a second section 24 disposed in the second recessed section 11, and a fluid outlet 19 disposed in the end wall 7 (See Figure 6). An external conduit 80 having a pressure activated one-way valve 82 is shown fluidically connecting the outlet 22 and inlet 23. An assay fluid supply conduit 84 provides fluid from a fluid reservoir (not shown) to an inlet divert valve 85, which is operable to fluidically connect the fluid supply conduit 84 with the fluid inlet 20 (to supply assay fluid to the plate) or to fluidically connect the fluid inlet 20 with a fluid recirculation conduit 86 to receive assay fluid that is being recirculated from the fluid outlet 19. The fluid recirculation conduit 86 also includes a pressure activated one-way valve 87. A second divert valve 88 is provided and operable to direct the assay fluid to waste or to the recirculation conduit.

In use, and referring to Figures 13 and 10, the sequential operation of the fluid control modules works to recirculate assay fluid across the wells via the first fluidic conduits. In a first step, the pins of the second fluid control module are depressed into engagement with the pumping wells of the second recessed section of the plate, and the pins of the first fluid control module are spaced from the plate, as shown in Figure 10. Due to the presence of the one-way valve 82 the assay fluid is forced into the fluid recirculation conduit 86 where it passes through the second pressure activated one-way valve 87. In a second step, the pins of the second fluid control module are retracted while the pins of the first fluid control module are depressed. This pushes assay fluid from the first section of the first fluidic conduits into the external conduit through the one-way valve 82 and simultaneously the retraction of the pins of the second fluid control module pulls assay fluid into the second section of the first fluidic conduits. Repeating this sequential operation effects the movement of assay fluid through the first fluidic conduits and wells and into the recirculation conduit, causing the recirculation of assay fluid across the wells of the plate.

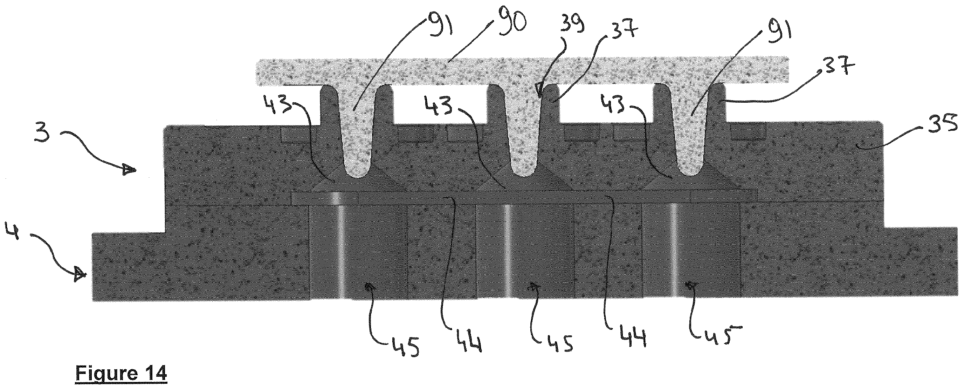

Referring to Figure 14, to fabricate the second plate 3, the first plate section 35 is formed by machining or casting, a mould 90 having nine well-shaped projections 91 is attached to the first plate section 35 with the projections 91 inserted into the open tops 39 of the wells 37. The first plate section 35 is then inverted and PDMS is added into the recess in the base of first plate section 35 (the recess can be seen in Figure 7B) where it fills the recess. The PDMS is then cured in an oven and forms the well imaging section 36 of the plate (light transparent and gas permeable) comprising the base 40 of each of the wells, plurality of frustoconical PDMS sections 43, and planar base 44. The base plate 4 is then attached to the second plate with the apertures 45 in register with the base of the wells which are viewable through the PDMS well imaging section.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. An assay plate assembly (1) to assay a 2D or 3-D cell culture model comprising
a first plate (2) comprising a plate body and a plurality of well-receiving apertures (14), and a fluidic conduit (12, 13) configured to provide an assay fluid to the well receiving apertures; and
a second plate (3) having a plurality of assay wells (37) each comprising a lower well portion (41) with a well base (40) disposed in the second plate and an upper well portion (38) having an open top (39) defined by an annular sidewall that projects above a top surface of the second plate,
wherein the first and second plates are configured to nest together to form the assay plate assembly in which the upper well portion (38) of each assay well (37) is disposed within a corresponding well-receiving aperture (14) of the first plate for receipt of fluid from the fluidic conduit (12, 13) through the open top of the cell culture model assay wells.

2. An assay plate assembly (1) according to Claim 1, in which the first plate (2) is re-usable and the second plate (3) is disposable.

3. An assay plate assembly (1) according to Claim 1 or 2, in which the lower part of the second plate (3) comprises a light transparent material configured to allow imaging of the assay wells (37) by an imaging device from underneath the wells.

4. An assay plate assembly (1) according to Claim 4, in which the lower part of the second plate comprises a plurality of well imaging sections comprising light transparent and gas permeable material, each having an upper section that forms the base (40) of the well and a lower section (43) that tapers outwardly towards a base of the second plate.

5. An assay plate assembly (1) according to any preceding Claim, in which the assay wells (37) comprise a sidewall that tapers inwardly from the open top (39) towards the base (40).

6. An assay plate assembly (1) according to any preceding Claim, in which the base (40) of the assay wells (37) has a diameter D1 and the open top (39) of the assay well has a diameter D2, wherein a ratio of diameter D1 to D2 is 3:6 to 5:6.

7. An assay plate assembly according to any preceding Claim, in which the assay wells (37) have a concave base (40).

8. An assay plate assembly according to any preceding Claim, in which the assay wells (37) have a volume of 5-100 µL.

9. An assay plate assembly according to any preceding Claim, in which the first plate (2) comprises a recess (10) formed in an upper surface thereof and a cover layer (15) comprising resiliently deformable material dimensioned to nest in the recess, in which the recess comprises an elongated groove and the fluidic conduit (12, 13) of the first plate is defined along at least a part of its length between the elongated groove and the cover layer (15).

10. An assay plate assembly according to any preceding Claim, in which the plurality of cell culture model assay wells (37) are arranged in a N x M grid assembly in which N and M are each, independently. a whole number greater than or equal to 3.

11. A system comprising:
an assay plate assembly (1) according to any of Claims 1 to 10;
a well imaging system configured to image one or more of the wells (37) of the assay plate assembly from underneath the assay plate assembly; and
a fluid control module (60) comprising at least one pin (62, 65) and an actuation system coupled to the at least one pin configured to move the pin between a first configuration in which the pin is spaced apart from the first plate (2) and a second configuration in which the pin is advanced into the fluidic conduit (12, 13) of the first plate to close the fluidic conduit.

12. A method of assaying a cell culture model that employs an assay plate assembly according to any of Claims 1 to 10 or a system according to Claim 11, the method comprising the steps of:
adding a cell culture model or precursor cells into a cell culture model assay well of the second plate;
coupling the second plate with the first plate such that the upper well portions of the cell culture model assay wells of the second plate are nested and fluidically sealed within corresponding well-receiving apertures of the first plate;
providing assay fluid containing a test compound to the cell culture model assay well containing the cell culture model or precursor cells through the fluidic conduit of the first plate; and
assaying the response of the cell culture model to the assay fluid containing the test compound.

13. A method according to Claim 12, in which the method comprises a step of growing at least one cell culture model in-situ within a cell culture model assay well after the assay plate assembly has been assembled, in which the assay fluid comprises a cell culture model culture medium.

14. A method according to any of Claims 12 to 14, in which the assay plate assembly comprises a column of wells fluidically connected in series in which at least one of the wells of the column contains a cell culture model having a first tissue or cell type and at least one other of the wells of the column contains a cell culture model having a second tissue or cell type.

15. A method according to Claim 12 or 15, the method comprises providing an assay fluid containing a test compound to a first well containing a cell culture model having a first tissue or cell type, and then providing fluid from the first well containing the test compound to a second well containing a cell culture model having a second tissue or cell type.
